# EUROPEAN PATENT APPLICATION

(11) **EP 1 716 815 A1**
(43) Date of publication of application: **02.11.2006**
(21) Application number: 06425282.8
(22) Date of filing: 28.04.2006
(51) Int. Cl.: A61B 17/88, B21D 7/06, A61B 17/80

(54) **Instrument for arch modelling of surgical plates for maxillo-facial surgery**

(30) Priority: 29.04.2005 IT MC20050037
(71) Applicant: PIERGIACOMI SUD - S.R.L., 63033 Monteprandone (AP) (IT)
(72) Inventor: Di Emidio, Paolo, 64010 Controguerra (TE) (IT)
(74) Representative: Baldi, Claudio

(57) **Abstract**

The present invention refers to an instrument used for arch modelling of surgical plates, which comprises a modelling head (2), with circular shape, supported at the end of a handle (3) and provided with a flat groove (4) laterally bordered by two edges (4a).

## Description

The present patent application for industrial invention refers to an instrument used for arch modelling of surgical plates that are normally used for connection and fixing of mandibular stumps following to resection imposed by the need to remove neoplastic infiltrations.

The 2-3% of all malignant tumours is represented by oral cavity tumours and 90% of these tumours are epidermis cancers characterised by marked aggressiveness.

Although the oral cavity can be easily accessed and inspected, unfortunately the large majority of these tumours is diagnosed tardily and, in most cases, laterocervical linphonodal metastases and mandibular infiltrations are present.

When neoplastic mandibular infiltration is diagnosed, the affected segment is normally resected.

In addition to comply with a series of parameters that guarantee a radical oncologic action, surgery must also provide for immediate and adequate anatomic and functional reconstruction of the surgical defect.

To that end, suitable surgical plates are available on the market to connect and fix the mandibular stumps.

The Italian patent application MC2001 U000029 by the same applicant discloses one of the said surgical plates, which consists in a sort of rectilinear titanium band, including a central section composed of a series of spheres connected by cylindrical arms and two ending sections, each of them composed of a series of rings connected by means of plates.

During the use and implantation of the said surgical plates, great difficulty is encountered when modelling the plates to give them a curvilinear direction that exactly corresponds to the profile of the missing bony segment, in order to ensure perfectly anatomical connection of the stumps. Most surgeons make use of modelling instruments that, however, are rather unsatisfactory, especially when modelling a plate used to replace the bony portion of the chin, i.e. the so-called interforaminal region, since this region has a strongly arched profile, basically shaped as a semicircle arch.

The purpose of the present invention is to devise a new instrument used for arch modelling of surgical plates normally used to connect and fix mandibular stumps, characterised by easy use, inexpensive production and total functionality.

The instrument of the invention comprises a modelling head supported at the end of a handle and consisting in a disk provided with a perimeter flat groove, whose width is such that it can house the surgical plate to be modelled, whose natural linear profile.

A small shelf protrudes along the border of the circular head and supports a pin, in orthogonal position with respect to the head, which is parallel and proximal to the bottom wall of the groove, so that the surgical plate is inserted edgeways in the thin space between the pin and the bottom wall, until it touches the radial shelf.

In order to insert the surgical plate, the two edges that laterally border the groove are interrupted for a short section astride the pin, so that the plate inserted in the said space practically adheres in a tangential way to the bottom wall of the circular groove and is ready to be bent against the said wall, on which the plate self-models, assuming the profile of a semicircle arch, the length of the arch being higher if the section of the groove embraced with the plate is longer.

For purposes of clarity the description of the device of the invention continues with reference to the enclosed drawings, which are intended for purposes of illustration only and not in a limiting sense, whereby:

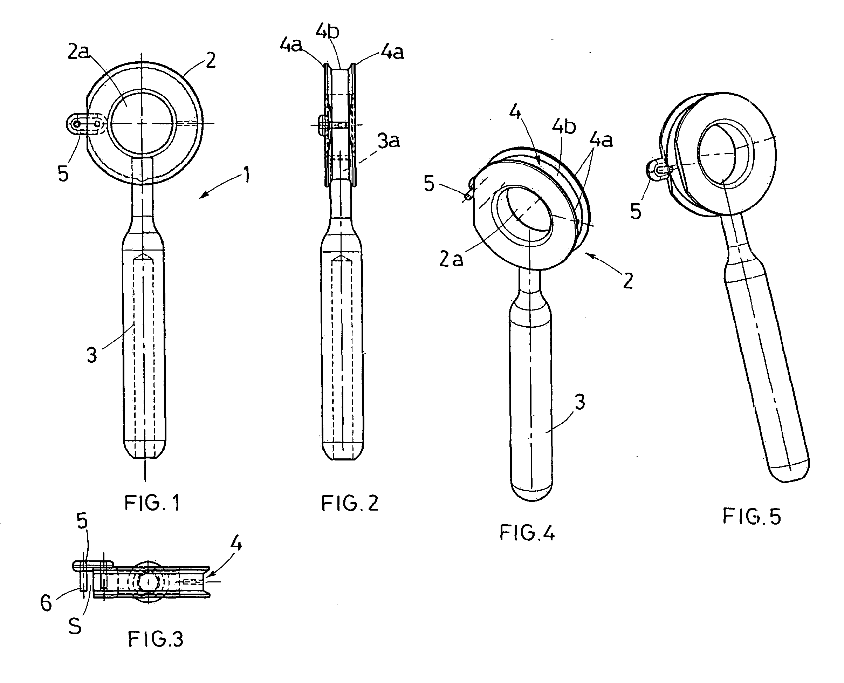

- figures 1, 2 and 3 are lateral, front and top views of the instrument of the invention, respectively.
- figures 4 and 5 are an axonometric view of the instrument of the invention seen from two different angles.
- figures 6 and 7 shows two phases of the bending process of a surgical plate performed with the instrument of the invention.
- figures 8 and 9 are a front and lateral view of a surgical plate of known type.

With reference to figures from 1 to 4, the instrument (1) of the invention comprises a modelling head (2), with circular shape, supported at the end of a handle (3), which preferably ends with a threaded shank section (3a) tightened into a radial hole provided on the external border of the head (2), preferably provided with a central lightening hole (2a).

The head (2) is provided with a flat groove (4) laterally bordered by two edges (4a) that are interrupted for a short section, which is provided with a small shelf (5) that protrudes with radial direction from the head (2).

More precisely, the shelf (5) is fixed on the rear side of the head (2) and supports a pin (6) in orthogonal position with respect to the head (2), which is parallel and proximal to the bottom wall (4b) of the groove (4).

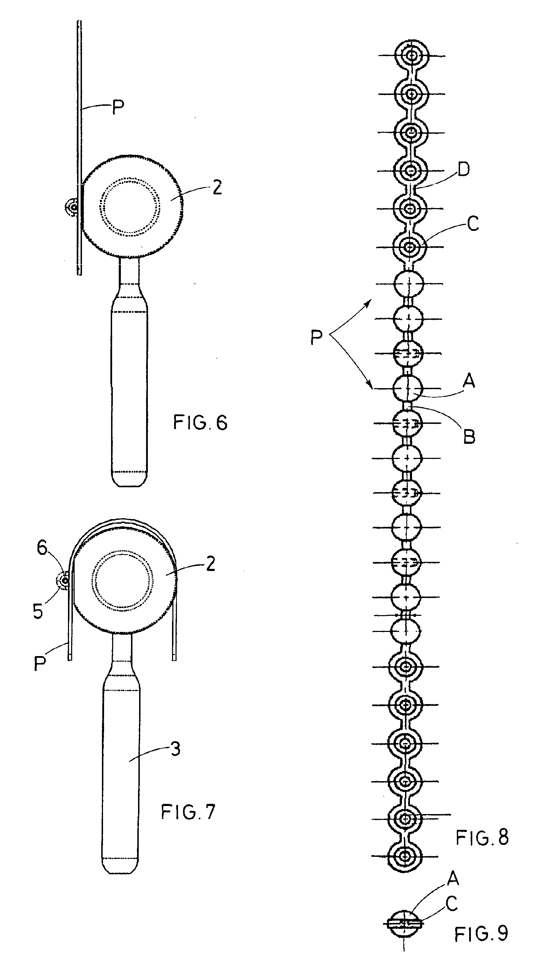

With reference to figures 8 and 9, the surgical plate (P) consists in a sort of rectilinear titanium band, including a central section composed of a series of spheres (A) connected by cylindrical arms (B) and two ending sections, each of them composed of a series of rings (C) connected by means of plates (D).

In order to model the plate (P), the plate is inserted edgeways in the thin space between the pin (6) and the bottom wall (4a), until it touches the radial shelf (5), as shown in fig. 6.

Attention is drawn on the fact that, due to the short interruption of the lateral edges (4a) the plate (P) inserted in the space (S) adheres with tangential direction against the bottom wall (4b) of the circular groove (4).

The plate (P) is bent against the bottom wall (4b) and inside the edges (4a), using the pin (6) as stop limit and anti-rollover means, as shown in fig. 7.

Evidently, the plate is self-modelled on the circular head (2), assuming the profile of a semicircle arch, with higher length in case of longer section of the groove (4) embraced with the plate.

Finally, it is noted that the pin (6) is positioned at 90° with respect to the handle (3).

Moreover, one of the ending sections of the plate (P) with the rings (C) connected by means of plates (D) is inserted in the space (S), while the section formed by the spheres (A) is designed to be bent against and inside the perimeter groove (4).

## Claims

1. Instrument for arch modelling of surgical plates for maxillo-facial surgery, **characterised in that** it comprises a modelling head (2), supported at the end of a handle (3) and consisting in a disk with a flat perimeter groove (4), whose bottom wall (4a) is limited by two lateral edges (4b) interrupted for a short section, which is provided with a small shelf (5) that protrudes with radial direction from the rear side of the head (3) and supports a pin (6) in orthogonal position with respect to the head (2), parallel and proximal to the bottom wall (4b) of the groove (4).

2. Instrument for arch modelling of surgical plates for maxillo-facial surgery, as defined in the preceding claim, **characterised in that** the pin (6) is positioned at 90° with respect to the handle (3).

3. Instrument for arch modelling of surgical plates for maxillo-facial surgery, as defined in one of the preceding claims, **characterised in that** the head (2) has a central lightening hole (2a).

4. Instrument for arch modelling of surgical plates for maxillo-facial surgery, as defined in one of the preceding claims, **characterised in that** the handle (3) ends with a threaded shank section (3a), tightened into a radial hole provided on the external border of the head (2).
